# EUROPEAN PATENT SPECIFICATION

(11) **EP 4 401 678 B1**
(45) Date of publication and mention of the grant of the patent: **29.07.2026**
(21) Application number: 22783014.8
(22) Date of filing: 14.09.2022
(51) Int. Cl.: A61F 2/24, F01L 9/10

(54) **TRANSCATHETER DELIVERY CATHETER ASSEMBLIES**
KATHETERANORDNUNGEN ZUR TRANSKATHETERFREISETZUNG
ENSEMBLES CATHÉTERS DE POSE TRANSCATHÉTER

(30) Priority: 17.09.2021 US 202163245298 P
(43) Date of publication of application: 24.07.2024
(73) Proprietor: Medtronic Inc., Minneapolis, MN 55432 (US)
(72) Inventor: WIEMEYER, Nathan B., Santa Rosa, California 95403 (US); MAUCH, Kevin M., Santa Rosa, California 95403 (US)
(74) Representative: Zimmermann & Partner Patentanwälte mbB
(86) International application number: PCT/IB2022/058662
(87) International publication number: WO 2023/042092

(56) References cited:
- EP-B1- 2 545 258
- US-A1- 2016 369 666
- US-A1- 2018 256 326
- US-A1- 2020 205 972

## Description

### FIELD

The present technology is generally related to transcatheter delivery catheter assemblies for the delivery and deployment of a prosthesis, such as a prosthetic heart valve.

### BACKGROUND

Diseased or otherwise deficient heart valves can be repaired or replaced with an implanted prosthetic heart valve. Conventionally, heart valve replacement surgery is an open-heart procedure conducted under general anesthesia, during which the heart is stopped and blood flow is controlled by a heart-lung bypass machine. Traditional open surgery inflicts significant patient trauma and discomfort, and exposes the patient to a number of potential risks, such as infection, stroke, renal failure, and adverse effects associated with the use of the heart-lung bypass machine, for example.

Due to the drawbacks of open-heart surgical procedures, there has been an increased interest in minimally invasive and percutaneous replacement of cardiac valves. With percutaneous transcatheter (or transluminal) techniques, a valve prosthesis is compacted for delivery in a transcatheter delivery device and then advanced, for example, through an opening in the femoral artery and through the descending aorta to the heart, where the prosthesis is then deployed in the annulus of the valve to be restored (e.g., the aortic valve annulus). Although transcatheter techniques have attained widespread acceptance with respect to the delivery of conventional stents to restore vessel patency, only mixed results have been realized with percutaneous delivery of the more complex prosthetic heart valve,

A transcatheter delivery device must often navigate through tortuous anatomy as it is tracked through the vasculature to the treatment site within the heart. The transcatheter delivery device may be navigated through various anatomical turns as it travels within the vasculature. For example, a transcatheter delivery device may be navigated through the sharp bend of the aortic arch.

The present disclosure addresses problems and limitations with the related art, US 2018/0256326 Al relates to delivery systems having a short capsule and a cinch mechanism.

### SUMMARY

The claimed subject-matter is defined in independent claim 1. Further aspects and preferred embodiments are defined in the dependent claims. The methods are provided for illustrative purposes.

The techniques of this disclosure generally relate to delivery catheter assemblies and transcatheter methods of delivering a prosthesis to a target site. In non-limiting examples, the prosthesis is a prosthetic heart valve and the target site is a native heart valve annulus. Such catheter assemblies include a capsule in which the prosthesis is sheathed during delivery. Various embodiments are configured to restrict movement of the capsule during deployment of the prosthesis to prevent assembly interference with patient anatomy and prevent the capsule from disengaging from the delivery catheter assembly.

In one aspect, the present disclosure provides delivery catheter assemblies including a catheter having a first portion and a second portion. The first portion extends along a first portion of a longitudinal axis and the second portion extending along a second portion of the longitudinal axis. The delivery catheter assemblies further include an elongate body having a first portion, a second portion, and an outer surface. At least the second portion of the elongate body is configured to fit within the catheter and to extend from at least the first portion of the catheter to the second portion of the catheter. The elongate body including a stop. The delivery catheter assemblies further include a capsule coupled to the second portion of the elongate body and movable along the longitudinal axis relative to the catheter. A fluid path is located along the longitudinal axis from the first portion of the catheter to a reservoir at least partly defined by the capsule. In various delivery catheter assemblies, the stop is operable to impede fluid flow through the fluid path and into the reservoir.

In another aspect, the disclosure provides methods of delivering and deploying a prosthesis. Such methods can include providing a delivery catheter assembly having a catheter including a first portion and a second portion. The first portion extends along a first portion of a longitudinal axis and the second portion extending along a second portion of the longitudinal axis. The delivery catheter assembly further including an elongate body having a first portion, a second portion, and an outer surface. At least the second portion of the elongate body being configured to fit within the catheter and to extend from at least the first portion of the catheter to the second portion of the catheter. The elongate body has a stop. The delivery catheter assembly also includes a capsule coupled to the second portion of the elongate body and movable along the longitudinal axis relative to the elongate body. A prosthesis is loaded within the capsule. Methods include delivering the capsule though a patient's vasculature to a treatment site and directing fluid along a fluid path over the elongate body and into a reservoir of the capsule to cause the capsule to advance with respect to the catheter. The methods include at least partially unsheathing the prosthesis until the stop restricts further movement of the capsule and prevents further fluid from being transferred from the catheter to the reservoir.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1 is a partial, cross-sectional view of a delivery catheter assembly.
FIG. 2 is a partial, cross-sectional view of the delivery catheter assembly of FIG. 1 in a fluid flow arrangement illustrating a fluid path within the delivery catheter assembly.
FIG. 3 is a partial, cross-sectional view of the delivery catheter assembly of FIGS. 1-2 in a fluid restricted arrangement in which a stop of the delivery catheter assembly is arranged to prevent the flow of fluid past the stop.
FIG. 4 is a partial, schematic illustration of a loaded prosthesis maintained within a capsule of the delivery catheter assembly of FIGS. 1-3.
FIG. 5 is a partial, schematic illustration of the delivery catheter assembly of FIG. 4 in which the prosthesis is partially deployed from the capsule.
FIG. 6 is a partial, schematic illustration of the delivery catheter assembly of FIGS. 3-4 in an arrangement in which the prosthesis (not shown for clarity) would be fully deployed and unsheathed by the capsule.
FIG. 7 is a side view of an alternate stop of the disclosure.
FIG. 8 is a side view of the stop of FIG. 7 engaged with a piston mount of the delivery catheter assembly of FIGS. 1-3 in the fluid restricted arrangement which would prevent fluid from passing over the stop and into the piston mount.
FIG. 9 is a side view of an alternate stop of the disclosure.
FIG. 10 is a side view of yet another stop of the disclosure that is incorporated into an elongate body of the delivery catheter assembly of FIGS. 1-3, for example.

### DETAILED DESCRIPTION

Specific embodiments of the present disclosure are now described with reference to the figures, wherein like reference numbers indicate identical or functionally similar elements. The terms "distal" and "proximal" are used in the following description with respect to a position or direction relative to the treating clinician. "Distal" or "distally" are a position distant from or in a direction away from the clinician. "Proximal" and "proximally" are a position near or in a direction toward the clinician.

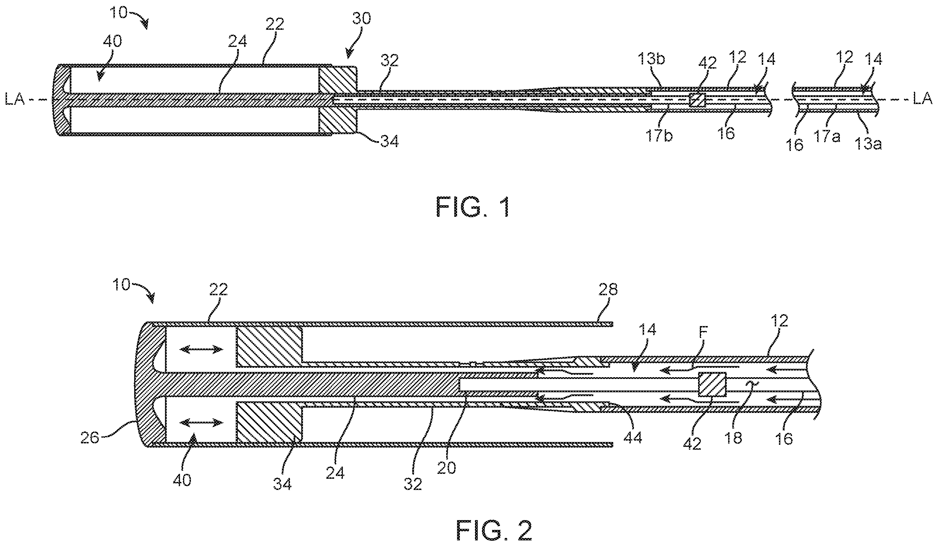

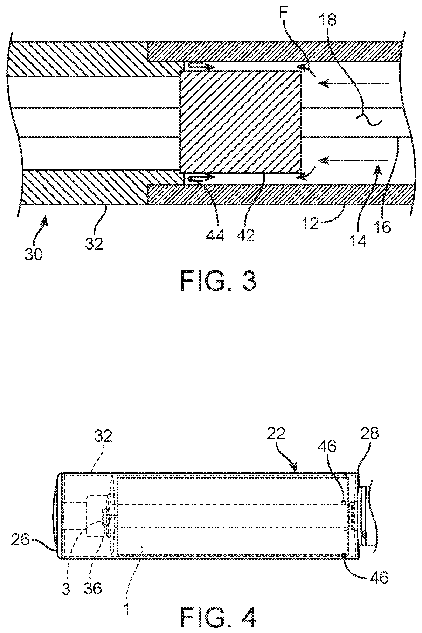

Referring to FIGS. 1-6, in general terms, one delivery catheter assembly 10 of the disclosure includes at least one catheter 12 and a lumen 14 defined by and/or extending through the catheter 12. The catheter 12 can include a first portion 13a and a second portion 13b each extending along a shared longitudinal axis LA of the catheter 12 and assembly 10. An elongate body 16 having a first portion 17a, a second portion 17b and an outer surface 18 extends at least partially within the lumen 14. In one example, at least the second portion 17b of the elongate body 16 is configured to fit within the catheter 12 and to extend from at least the first portion 13a of the catheter 12 to the second portion 13b of the catheter 12, within lumen 14. A tip 20 of the elongate body 16 can be secured to a support shaft 24 of a capsule 22 configured to selectively sheathe a prosthesis 1 (schematically shown in FIGS. 4-5). The capsule 22 can have a closed end 26 and an open end 28 out which the prosthesis 1 can be deployed. In some embodiments, the closed end 26 is positioned distal with respect to the open end 28. To further support the prosthesis 1 while at least partially maintained within the capsule 22, the delivery catheter assembly 10 can include a piston assembly 30 slidably positioned at least partially within the capsule 22. The piston assembly 30 can be positioned over one or more of the support shaft 24 and the elongate body 16. The piston assembly 30 can be configured to selectively maintain and support the prosthesis 1 in any known manner. In one non-limiting example, the piston assembly 30 includes a piston mount 32 and a piston head 34. In one non-limiting example, the piston head 34 includes one or more slots 36 (e.g., in the shape of a T) configured to receive corresponding features 3 on the prosthesis 1 as is perhaps best shown in FIGS. 4-5. In various examples, the piston head 34 is integrally formed with the piston mount 32 or the piston head 34 and in other various examples, the piston head 34 is a separate component that is mounted onto the piston mount 32.

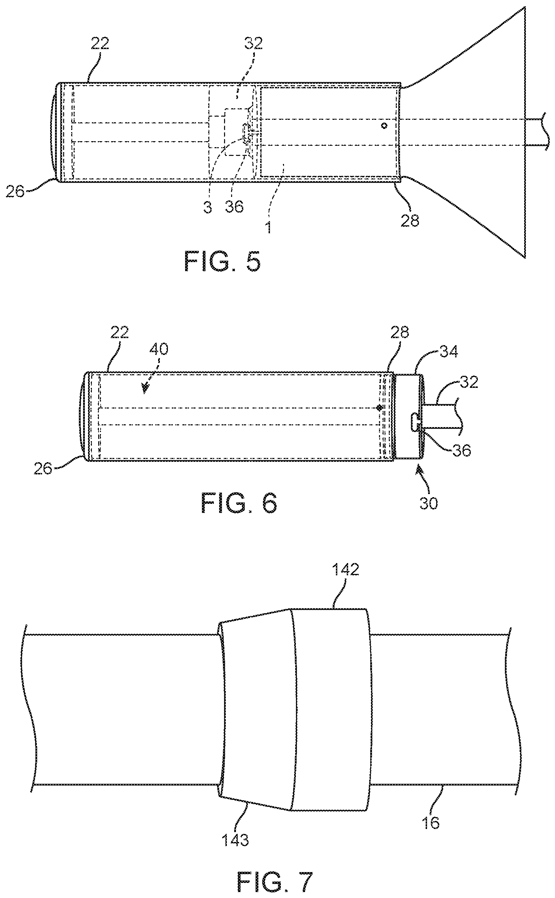

As perhaps best shown in FIGS. 4-6, unsheathing of the prosthesis 1 by the capsule 22 from the fully loaded arrangement of FIG. 4, to a partially deployed arrangement of FIG. 5 to a fully deployed arrangement of FIG. 6, is controlled by advancing the capsule 22 with respect to the catheter 12, piston assembly 30 and prosthesis 1 maintained thereon. In one example, advancement of the capsule 22 with respect to the catheter 12 to unsheathe the prosthesis 1 can be controlled by injecting fluid F into a reservoir 40 adjacent the closed end 26 of the capsule 22 to push the closed end 26 away from the piston assembly 30. In one example, fluid F flows along a fluid path within the lumen 14, over the outer surface 18 of the elongate body 16, between the piston head 34 and the support shaft 24 and then into the reservoir 40 defined by a plurality of walls or surfaces of at least both of the capsule 22 and piston assembly 30. In the illustrated example, the reservoir 40 is formed by at least the capsule 22 and the piston head 34. Optionally, the piston head 34 can include a seal (not shown), such as an annular ring or the like to assist in maintaining fluid within the reservoir 40. As fluid F fills the reservoir 40, the capsule 22 is pushed away from the piston assembly 30. As the capsule 22 is connected to the elongate body 16, advancement or movement of the capsule 22 will correspond to equal advancement or movement of the elongate body 16. In one example, distal advancement of the capsule 22 will correspond to equal distal advancement of the elongate body 16. To prevent overextension or restrict movement of the capsule 22, the elongate body 16 can include a stop 42, wherein the position of the stop 42 with respect to the catheter 12 indicates whether the delivery catheter assembly 10 is in a fluid flow arrangement (FIG. 2) in which fluid F can flow from a location within the lumen 14 to the reservoir 40 within the capsule 22 or a fluid restricted arrangement (FIG. 3) in which the position of the stop 42 prevents the flow of fluid F between the location within the lumen 14 and to the reservoir 40 within the capsule 22. In the fluid restricted arrangement, a maximum advancement of the capsule 22 with respect to the piston assembly 30 has been achieved. In general terms, the stop 42 is operable to impede fluid F flow through the fluid path, into the reservoir 40.

In one non-limiting example, the stop 42 is a portion of the elongate body 16 having an increased outer diameter such that the stop 42 forms a fluid-tight seal when distally advanced to contact a corresponding element of the lumen 14 or delivery catheter assembly 10. In the illustrated example, the stop 42 is configured to form a seal when the stop 42 contacts the piston mount 32 of the piston assembly 30, blocking fluid F from moving past the stop 42 in either direction along the fluid path. In various embodiments, the piston mount 32 may have a smaller inner diameter as compared to an inner diameter of the lumen 14 such that the junction of the piston mount 32 and the lumen 14 form a ridge 44 that the stop 42 will abut in the fluid restricted arrangement. Optionally, the capsule 22 can also include one or more vent holes 46 at the open end 28 of the capsule 22 to serve as a backup means for releasing fluid F from the reservoir 40 to restrict distal movement of the capsule 22 to further maintain connection between the capsule 22 and the piston assembly 30. Generally, the venting of fluid F outside the delivery catheter assembly 10 or capsule 22 is undesirable as the fluid F reduces or obstructs visualization of the deployment of the prosthesis 1 when viewed using ultrasound echocardiography. The release of fluid F may appear as cavitation, which can be distracting to the attending physician. In view of the present disclosure, it will be understood that the stop 42 can be positioned at various locations along the elongate body 16 and that the lumen 14 or other elements of the delivery catheter assembly 10 can be configured to interface and form a seal with the stop 42 when the stop reaches a longitudinal location corresponding with a desired limit of distal movement of the capsule 22. Delivery catheter assemblies having a stop (e.g., stop 42 or any other stop disclosed herein) that is positioned proximal with respect to the piston head 34 or other location where the prosthesis 1 is secured to the delivery catheter assembly 10 are beneficial in that the stop, which has an increased outer diameter as compared to the elongate body 16, piston mount 32, and support shaft 24, will not affect (i.e. enlarge) the crimping profile of the prosthesis 1.

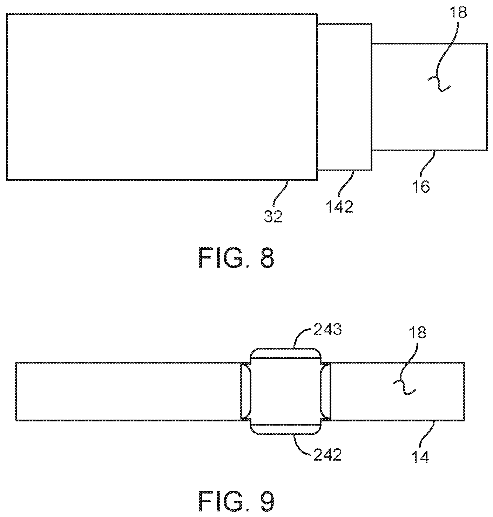

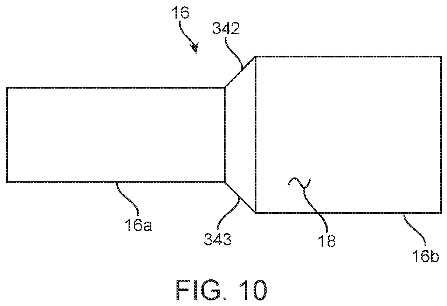

Stops of the disclosure can take a variety of configurations to achieve restriction or impedance of fluid flow within the lumen 14. In the broadest sense, the stops of the disclosure are configured to interact with the lumen 14, piston assembly 30 or another portion of the delivery catheter assembly 10 to both prevent further movement of the elongate body 16 away from the catheter 12 and also restrict fluid F flow past the stop and into the capsule 22 to prevent the capsule from further advancement. In some examples, movement of the capsule 22 can be described as being restricted in a distal direction and that the stops of the disclosure prevent fluid F from moving distally from the lumen 14, past the stop. In the example of FIGS. 1-3, the stop 42 is configured to abut or interface with a proximal end of the piston mount 32 preventing further advancement of the capsule 22. In the example of FIGS. 7-8, a stop 142 can include a tapered end 143 configured to engage and at least partially be positioned within the piston mount 32 to form a fluid seal. In some examples, the end 143 can be described as a distal end. The stop 142 can be connected to the outer surface 18 of the elongate body 16 or can be integrally formed with the elongate body 16. In some alternate examples, a stop 242 can include a crimp 243 in a band secured on the outer surface 18 of the elongate body 16. Alternatively, the crimp 243 can be formed in the elongate body 16 itself. The band can optionally be made of metal or a polymer, for example. In yet another example, the stop 242 can include welded beads positioned around the outer surface 18 of the elongate body 16 to increase an outer diameter of the elongate body 16 at the stop 242. As shown in FIG. 10, in yet another example, a stop 342 can alternatively include a tapered portion 343 on the outer surface 18 of the elongate body 16 such that a distal or first section 16a of the elongate body 16, distal to the stop 342, has a smaller outer diameter as compared to a proximal or second section 16b of the elongate body 16 proximal to the stop 342 that allows the first section 16a of the elongate body 16 to pass through the piston mount 32 but will not allow the stop 342 to pass entirely through the piston mount 32.

Aspects of the disclosure are beneficial for use with cardiac prostheses (e.g., prosthesis 1), prosthetic heart valves and heart valve repair methods including the implantation of a prosthetic heart valve, particularly, prosthetic heart valves delivered via transcatheter procedure. As referred to herein, prosthetic heart valves ("prosthetic valves" or "prosthesis") can include a bioprosthetic heart valve having tissue leaflets or a synthetic heart valve having polymeric, metallic or tissue-engineered leaflets, and can be specifically configured for replacing valves of the human heart. In one non-limiting example, the valve of the human heart is an aortic valve. The prosthetic valves of the present disclosure may be self-expandable, balloon expandable and/or mechanically expandable or combinations thereof. In general terms, the prosthetic valves of the present disclosure include a stent or stent frame having an internal lumen maintaining a valve structure (tissue or synthetic), with the stent frame having a normal, expanded condition or arrangement and collapsible to a compressed condition or arrangement for loading within the delivery catheter assembly. For example, the stents or stent frames are support structures that comprise a number of struts or wire segments arranged relative to each other to provide a desired compressibility and strength to the prosthetic valve. The struts or wire segments are arranged such that they are capable of self-transitioning from, or being forced from, a compressed or collapsed arrangement to a normal, radially expanded arrangement. The struts or wire segments can optionally be formed from a shape memory material, such as a nickel titanium alloy (e.g., nitinol). The stent frame can be laser-cut from a single piece of material, or can be assembled from a number of discrete components. Other prostheses delivered via transcatheter procedure are also envisioned as being suitable for use with the delivery catheter assemblies and methods of the disclosure as well.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

## Claims

1. A delivery catheter assembly (10) comprising:
a catheter (12) including a first portion (13a) and a second portion (13b), the first portion extending along a first portion of a longitudinal axis (LA) and the second portion extending along a second portion of the longitudinal axis;
an elongate body (16) having a first portion (17a), a second portion (17b), and an outer surface (18), at least the second portion of the elongate body configured to fit within the catheter and to extend from at least the first portion of the catheter to the second portion of the catheter, the elongate body including a stop (42);
a capsule (22) coupled to the second portion of the elongate body and movable along the longitudinal axis relative to the catheter; and
a fluid path along the longitudinal axis from the first portion of the catheter to a reservoir (40) at least partly defined by the capsule;
wherein the stop is operable to impede fluid flow through the fluid path and into the reservoir.

2. The delivery catheter assembly of claim 1, wherein the delivery catheter assembly includes a piston assembly (30) at least partially contained within the capsule and the fluid path is at least partially defined by at least a portion of the piston assembly.

3. The delivery catheter assembly of claim 2, wherein the reservoir comprises walls comprising a capsule wall and at least a portion of the piston assembly.

4. The delivery catheter assembly of claim 3, the piston assembly including a piston mount (32) and a piston head (34), wherein the piston mount interfaces with the stop to prevent the piston assembly from longitudinally moving in at least one direction.

5. The delivery catheter assembly of claim 4, wherein the elongate body is partially positioned within the piston mount, or
wherein the piston head includes at least one slot configured to receive and maintain a prosthesis when the prosthesis is positioned within the capsule.

6. The delivery catheter assembly of claim 1, wherein the stop is integrally formed with the elongate body, or
wherein the stop is connected to the outer surface of the elongate body.

7. The delivery catheter assembly of claim 1, wherein the capsule includes a support shaft (24) that is secured to an end of the elongate body; wherein movement of the capsule translates to movement of the elongate body.

8. The delivery catheter assembly of claim 1, wherein the stop has a tapered end (143).

9. A system for deploying a prosthesis, the system comprising:
the delivery catheter assembly of claim 1, and
a prosthesis loaded within the capsule;
wherein the system is configured to deliver the capsule though a patient's vasculature to a treatment site; and
wherein when fluid is directed along the fluid path over the elongate body and into the reservoir of the capsule, the system is configured to cause the capsule to advance with respect to the catheter, at least partially unsheathing the prosthesis, until the stop restricts further movement of the capsule and prevents further fluid from being transferred from the catheter to the reservoir.

10. The system of claim 9, wherein the prosthesis is a prosthetic heart valve, or
wherein the stop is positioned within the catheter when the stop prevents further fluid from being transferred from the catheter to the reservoir.

11. The system of claim 9, wherein the delivery catheter assembly further includes a piston assembly including a piston mount; wherein the system is configured to restrict movement of the capsule with respect to an end of the catheter when the stop contacts the piston mount.

12. The system of claim 11, wherein the piston assembly includes a piston head extending from the piston mount, wherein the piston head is configured to retain the prosthesis when the prosthesis is loaded within the capsule, in particular,
wherein the capsule includes a support shaft connected to an end of the elongate body; wherein the fluid path is partially located between the piston assembly and the support shaft.

13. The system of claim 9, wherein the stop is connected to the outer surface of the elongate body, or
wherein the stop is integrally formed with the elongate body.

14. The system of claim 9, wherein the stop is configured to advance as the capsule advances.

15. The system of claim 9, wherein the system is configured to deploy the prosthesis from a proximal end of the capsule.

## Patentansprüche

1. Zuführkatheterbaugruppe (10), umfassend:
einen Katheter (12), der einen ersten Abschnitt (13a) und einen zweiten Abschnitt (13b) aufweist, wobei sich der erste Abschnitt entlang eines ersten Abschnitts einer Längsachse (LA) erstreckt und sich der zweite Abschnitt entlang eines zweiten Abschnitts der Längsachse erstreckt,
einen länglichen Körper (16) mit einem ersten Abschnitt (17a), einem zweiten Abschnitt (17b) und einer Außenfläche (18), wobei mindestens der zweite Abschnitt des länglichen Körpers dazu ausgestaltet ist, in den Katheter zu passen und sich von mindestens dem ersten Abschnitt des Katheters zu dem zweiten Abschnitt des Katheters zu erstrecken, wobei der längliche Körper einen Anschlag (42) aufweist,
eine Kapsel (22), die an den zweiten Abschnitt des länglichen Körpers gekoppelt und entlang der Längsachse relativ zu dem Katheter beweglich ist, und
einen Fluidpfad entlang der Längsachse von dem ersten Abschnitt des Katheters zu einem Reservoir (40), das mindestens teilweise durch die Kapsel definiert ist,
wobei der Anschlag dahingehend betätigbar ist, Fluidströmung durch den Fluidpfad und in das Reservoir zu verhindern.

2. Zuführkatheterbaugruppe nach Anspruch 1, wobei die Zuführkatheterbaugruppe eine Kolbenbaugruppe (30) aufweist, die mindestens teilweise in der Kapsel enthalten ist, und der Fluidweg mindestens teilweise durch mindestens einen Abschnitt der Kolbenbaugruppe definiert ist.

3. Zuführkatheterbaugruppe nach Anspruch 2, wobei das Reservoir Wände umfasst, die eine Kapselwand und mindestens einen Abschnitt der Kolbenbaugruppe umfassen.

4. Zuführkatheterbaugruppe nach Anspruch 3, wobei die Kolbenbaugruppe eine Kolbenhalterung (32) und einen Kolbenkopf (34) aufweist, wobei die Kolbenhalterung mit dem Anschlag koppelt, um zu verhindern, dass sich die Kolbenbaugruppe in Längsrichtung in mindestens eine Richtung bewegt.

5. Zuführkatheterbaugruppe nach Anspruch 4, wobei der längliche Körper teilweise in der Kolbenhalterung positioniert ist oder
wobei der Kolbenkopf mindestens einen Schlitz aufweist, der zum Aufnehmen und Halten einer Prothese ausgestaltet ist, wenn die Prothese in der Kapsel positioniert ist.

6. Zuführkatheterbaugruppe nach Anspruch 1, wobei der Anschlag integral mit dem länglichen Körper ausgebildet ist oder
wobei der Anschlag mit der Außenfläche des länglichen Körpers verbunden ist.

7. Zuführkatheterbaugruppe nach Anspruch 1, wobei die Kapsel einen Stützschaft (24) aufweist, der an einem Ende des länglichen Körpers befestigt ist, wobei eine Bewegung der Kapsel in eine Bewegung des länglichen Körpers umgesetzt wird.

8. Zuführkatheterbaugruppe nach Anspruch 1, wobei der Anschlag ein verjüngtes Ende (143) aufweist.

9. System zum Ausbringen einer Prothese, wobei das System Folgendes umfasst:
die Zuführkatheterbaugruppe nach Anspruch 1 und
eine in die Kapsel geladene Prothese,
wobei das System zum Zuführen der Kapsel durch das Gefäßsystem eines Patienten an eine Behandlungsstelle ausgestaltet ist und
wobei das System dazu ausgestaltet ist zu veranlassen, dass sich die Kapsel in Bezug auf den Katheter vorwärtsbewegt,
wenn Fluid entlang des Fluidpfads über den länglichen Körper und in das Reservoir der Kapsel geleitet wird, wobei die Prothese mindestens teilweise entmantelt wird, bis der Anschlag die weitere Bewegung der Kapsel einschränkt und verhindert, dass weiteres Fluid von dem Katheter zu dem Reservoir übertragen wird.

10. System nach Anspruch 9, wobei die Prothese eine Herzklappenprothese ist oder
wobei der Anschlag in dem Katheter positioniert ist, wenn der Anschlag ein Übertragen von weiterem Fluid von dem Katheter zu dem Reservoir verhindert.

11. System nach Anspruch 9, wobei die Zuführkatheterbaugruppe ferner eine Kolbenbaugruppe aufweist, die eine Kolbenhalterung aufweist, wobei das System dazu ausgestaltet ist, die Bewegung der Kapsel in Bezug auf ein Ende des Katheters einzuschränken, wenn der Anschlag die Kolbenhalterung kontaktiert.

12. System nach Anspruch 11, wobei die Kolbenbaugruppe einen Kolbenkopf aufweist, der sich von der Kolbenhalterung erstreckt, wobei der Kolbenkopf dazu ausgestaltet ist, die Prothese zu halten, wenn die Prothese in die Kapsel geladen wird, insbesondere
wobei die Kapsel einen Stützschaft aufweist, der mit einem Ende des länglichen Körpers verbunden ist, wobei sich der Fluidpfad teilweise zwischen der Kolbenbaugruppe und dem Stützschaft befindet.

13. System nach Anspruch 9, wobei der Anschlag mit der Außenfläche des länglichen Körpers verbunden ist oder wobei der Anschlag integral mit dem länglichen Körper ausgebildet ist.

14. System nach Anspruch 9, wobei der Anschlag dazu ausgestaltet ist, sich vorwärts zu bewegen, wenn sich die Kapsel vorwärts bewegt.

15. System nach Anspruch 9, wobei das System dazu ausgestaltet ist, die Prothese von einem proximalen Ende der Kapsel auszubringen.

## Revendications

1. Ensemble cathéter de pose (10) comprenant :
un cathéter (12) comprenant une première partie (13a) et une seconde partie (13b), la première partie s'étendant le long d'une première partie d'un axe longitudinal (LA) et la seconde partie s'étendant le long d'une seconde partie de l'axe longitudinal ;
un corps allongé (16) ayant une première partie (17a), une seconde partie (17b) et une surface extérieure (18), au moins la seconde partie du corps allongé étant conçue pour pouvoir être insérée à l'intérieur du cathéter et s'étendre au moins de la première partie du cathéter à la seconde partie du cathéter, le corps allongé comprenant un élément d'arrêt (42) ;
une capsule (22) accouplée à la seconde partie du corps allongé et mobile le long de l'axe longitudinal par rapport au cathéter ; et
une voie de fluide le long de l'axe longitudinal de la première partie du cathéter à un réservoir (40) défini au moins partiellement par la capsule ;
dans lequel l'élément d'arrêt est actionnable pour empêcher l'écoulement de fluide à travers la voie de fluide et dans le réservoir.

2. Ensemble cathéter de pose selon la revendication 1, l'ensemble cathéter de pose comprenant un ensemble piston (30) au moins partiellement logé à l'intérieur de la capsule et la voie de fluide étant au moins partiellement définie par au moins une partie de l'ensemble piston.

3. Ensemble cathéter de pose selon la revendication 2, dans lequel le réservoir comprend des parois comprenant une paroi de capsule et au moins une partie de l'ensemble piston.

4. Ensemble cathéter de pose selon la revendication 3, l'ensemble piston comprenant un support de piston (32) et une tête de piston (34), dans lequel le support de piston forme une interface avec l'élément d'arrêt pour empêcher l'ensemble piston de se déplacer longitudinalement dans au moins une direction.

5. Ensemble cathéter de pose selon la revendication 4, dans lequel le corps allongé est partiellement positionné à l'intérieur du support de piston, ou
dans lequel la tête de piston comprend au moins une fente conçue pour recevoir et maintenir une prothèse lorsque la prothèse est positionnée à l'intérieur de la capsule.

6. Ensemble cathéter de pose selon la revendication 1, dans lequel l'élément d'arrêt est formé d'un seul tenant avec le corps allongé, ou
dans lequel l'élément d'arrêt est raccordé à la surface extérieure du corps allongé.

7. Ensemble cathéter de pose selon la revendication 1, dans lequel la capsule comprend une tige de support (24) qui est fixée à une extrémité du corps allongé ; dans lequel le mouvement de la capsule se traduit par un mouvement du corps allongé.

8. Ensemble cathéter de pose selon la revendication 1, dans lequel l'élément d'arrêt a une extrémité fuselée (143).

9. Système de mise en place d'une prothèse, le système comprenant :
l'ensemble cathéter de pose selon la revendication 1, et une prothèse chargée à l'intérieur de la capsule ;
le système étant conçu pour acheminer la capsule à travers le système vasculaire d'un patient jusqu'à un site de traitement ; et
le système étant conçu, lorsque du fluide est dirigé le long de la voie de fluide sur le corps allongé et dans le réservoir de la capsule,
pour amener la capsule à avancer par rapport au cathéter, de façon à dégainer au moins partiellement la prothèse, jusqu'à ce que l'élément d'arrêt empêche un mouvement plus avant de la capsule et empêche que davantage de fluide soit transféré du cathéter au réservoir.

10. Système selon la revendication 9, dans lequel la prothèse est une valvule cardiaque prothétique, ou
dans lequel l'élément d'arrêt est positionné à l'intérieur du cathéter lorsque l'élément d'arrêt empêche que davantage de fluide soit transféré du cathéter au réservoir.

11. Système selon la revendication 9, dans lequel l'ensemble cathéter de pose comprend en outre un ensemble piston comprenant un support de piston ; le système étant conçu pour limiter le mouvement de la capsule par rapport à une extrémité du cathéter lorsque l'élément d'arrêt entre en contact avec le support de piston.

12. Système selon la revendication 11, dans lequel l'ensemble piston comprend une tête de piston s'étendant à partir du support de piston, dans lequel la tête de piston est conçue pour retenir la prothèse lorsque la prothèse est chargée à l'intérieur de la capsule, en particulier,
dans lequel la capsule comprend une tige de support reliée à une extrémité du corps allongé ; dans lequel la voie de fluide est partiellement située entre l'ensemble piston et la tige de support.

13. Système selon la revendication 9, dans lequel l'élément d'arrêt est relié à la surface extérieure du corps allongé, ou
dans lequel l'élément d'arrêt est formé d'un seul tenant avec le corps allongé.

14. Système selon la revendication 9, dans lequel l'élément d'arrêt est conçu pour avancer à mesure que la capsule avance.

15. Système selon la revendication 9, le système étant conçu pour déployer la prothèse à partir d'une extrémité proximale de la capsule.
